# EUROPEAN PATENT APPLICATION

(11) **EP 4 434 529 A1**
(43) Date of publication of application: **25.09.2024**
(21) Application number: 23382262.6
(22) Date of filing: 21.03.2023
(51) Int. Cl.: A61K 35/28, A61P 11/00

(54) **PHARMACEUTICAL COMPOSITION FOR PREVENTING OR TREATING BRONCHOPULMONARY DYSPLASIA BY THE INTRAVENOUS ROUTE IN REPEATED DOSES**

(71) Applicant: Fundación para la Investigación Biomédica del Hospital Universitario Ramón y Cajal, 28034 Madrid (ES); Fundación Para La Investigación Biomédica Del Hospital Infantil Universitario Niño Jesús, 28009 Madrid (ES)
(72) Inventor: DEL CERRO MARÍN, María Jesús, 28034 Madrid (ES); ÁLVAREZ FUENTE, María, 28034 Madrid (ES); RAMIREZ ORELLANA, Manuel, 28009 MADRID (ES); MELEN FRAJLICH, Gustavo Javier, 28009 MADRID (ES)
(74) Representative: Hoffmann Eitle

(57) **Abstract**

The present invention pertains to the medical field. In particular, to a pharmaceutical composition comprising an effective amount of isolated human mesenchymal stem cells (MSCs) in an acceptable pharmaceutical liquid medium for injection, for use in a method of preventing or treating a human subject having or at risk of developing bronchopulmonary dysplasia.

## Description

### Technical field

The present invention pertains to the medical field. In particular, to a pharmaceutical composition comprising an effective amount of isolated human mesenchymal stem cells (MSCs) in an acceptable pharmaceutical liquid medium for injection, for use in a method of preventing or treating a human subject having or at risk of developing bronchopulmonary dysplasia.

### Background art

Despite major advances in neonatal medicine, limited progress has been made in reducing the rates of bronchopulmonary dysplasia (BPD). In fact, BPD prevalence is increasing due to improved survival of extremely low gestational age newborn (ELGAN) infants (<28 weeks GA) and remains the leading cause of morbimortality in this population (1).

The pathophysiology of BPD is complex, with inflammation, oxidative stress, abnormal vasculogenesis and impaired lung repair as main actors (2).

Human Umbilical Cord-derived Mesenchymal Stem Cells (UC-MSC) have been able to prevent or ameliorate BPD in several pre-clinical studies (3-5), mainly due to the paracrine effects of MSC-derived humoral factors capacity to modulate macrophage actions (3) and due to epigenetic and phenotypic reprogramming of myeloid cells (6).

Lately, BPD mouse preclinical models evaluating a treatment strategy for established BPD, have shown that repeated doses of MSCs were superior to a single dose, attenuating alveolar injury and improving vessel density (9). Nevertheless, the translation of these preclinical data to human care should be put under scrutiny (10).

The first-in-human clinical trial with MSC as treatment for BPD was performed as a phase I dose-escalation trial using umbilical cord blood-derived MSCs, administered intratracheally (11). In this trial, the authors reported safety and a trend towards a beneficial effect in the MSC treated subgroup with a lower gestational age. These same investigators published a phase II trial in ELGAN with a trend to BPD rate improvement in the subgroup with lower GA (24-26 weeks) (12).

For the present invention, the authors, in their first off-label experience, have explored the feasibility and safety of intravenous, repeated and increasing doses of UC-MSCs in two ELGAN infants with severe and advanced BPD (13). We observed that the intravenous administration of UC-MSC was associated with a decrease in serum inflammatory biomarkers. In the necropsy study, we excluded engraftment of the MSCs in the recipient lung and observed severe fibrosis in the lungs of infants with chronic BPD after months on mechanical ventilation (13).

After this preliminary clinical experience, we designed and performed a phase I clinical trial testing feasibility and safety of intravenous, repeated doses of UC-MSCs in ELGAN infants administered in the first weeks of life.

### Description of embodiments

In the present invention, we herein described that the Intravenous administration of 5 × 10⁶ cells/kg (total dose 15 × 10⁶ cells/kg) of allogenic UC-MSCs administered once a week for three weeks is not associated with immediate SAEs or signs of toxicity in our cohort of ELGAN infants at high risk for developing BPD. By contrast, in the examples a significant efficacy of this therapy is made plausible. It is important to note that, for the present invention, the way in which the Intravenous administration of each dose of 5 × 10⁶ cells/kg of allogenic UC-MSCs is carried out, constitutes a fundamental aspect of the present invention. In this sense, because of the type of patients to be treated (ELGAN infants at high risk for developing BPD), in the present invention, each dose of 5 × 10⁶ cells/kg of allogenic UC-MSCs is in turn administered in repeated sub doses of about one million MSCs/ml. Each of these sub dosages are packaged directly into pre-filled syringes of 1 ml each, wherein the number of pre-filled syringes depends on the weight of the patient. Each of these pre-filled syringes are prepared to be used for the manual injection of the MScs via the intravenous route. In particular, after being extracted from the packaging, each of these pre-filled syringes should be preferably tempered and shaken gently by hand, until visually verifying that there are no aggregates of MSCs. Each of these sub dosages should be sequentially administered, preferably using a time of 1-2 minutes for the infusion of the contents of each pre-filled syringe. The different syringes are preferably thus injected successively, using a total time of between 5 and 10 minutes to inject all of them (the number of syringes and the total time needed to inject about 5 × 10⁶ cells/kg depends on the weight of the patient).

This invention opens lines of research with UC-MSC as a therapy for preterm infants with high risk for BPD by using an Intravenous or peripheral administration. In this sense, selecting the ideal route for UC-MSC transplantation or administration is a relevant issue. Intratracheal administration could have the advantage of direct effect on the lung, but it would not be feasible in those patients that are on non-invasive ventilation. This is important considering that the management of patients with non-invasive ventilation is increasing (23). In fact, prior to performing the clinical trial that has given rise to the present invention, the inventors conducted a preclinical study in which UC-MSC were intravenously administered to a murine model (24). This study showed that most of the injected cells reach the lung ("first pass effect") and stayed there for 4 to 7 days, disappearing afterwards. Therefore, apparently UC-MSC administered intravenously reaches the target organ, where the cells perform their therapeutic effect, and then disappear, reinforcing the thesis of a paracrine effect. Intranasal administration of MSCs has also been effective in a preclinical model of BDP (25). Both intravenous and intranasal administration may reach organs other than the lung (brain, kidney, etc.) which renders also interesting if we aim to target other organs with this therapy.

To date, no actual data can predict, at an early stage, which patients will develop the worse outcomes of BPD, which makes it difficult to determine the optimal timing for UC-MSC administration. Nevertheless, a risk score study (26) showed that being on IMV at postnatal day + 7 increased the risk of moderate/severe BPD 27 times. Jung et al. proved an up to 11-fold increase in the risk of developing severe BPD in those patients with RSS > 3 at postnatal day 14 in a population of 138 ELGAN (27). Also, in experimental models of BPD related with chorioamnionitis, prenatal administration of exosomes was able to preserve lung development (28). Considering all these trials, an earlier intervention, before lung damage and fibrosis are established, would probably be one of the keys to diminish risk of severe BPD (29).

In the present invention, we do not only demonstrate that the allogenic UC-MSCs administration once a week for three weeks or more is not associated with immediate SAEs or signs of toxicity in our cohort of ELGAN infants at high risk for developing BPD but, remarkably, that such administration produced no mortality.

In conclusion, the present invention shows the feasibility and safety of repeated intravenously UC-MSCs administration in ELGAN infants at high risk of BPD at short and midterm.

Therefore, a first aspect of the invention refers to a pharmaceutical composition a pharmaceutical composition comprising an effective amount of isolated human mesenchymal stem cells (MSC) in an acceptable pharmaceutical liquid medium for injection, for use in a method of preventing or treating a human subject having or at risk of developing bronchopulmonary dysplasia, wherein the human subject is less than four weeks old, has a birth weight ≤1,250 grams and <32 weeks gestational age (GA), preferably <28 weeks gestational age (GA), and has high risk of developing BPD, and wherein the pharmaceutical composition is administered intravenously to the subject in repeated doses over a period of several weeks (e.g., 1-2 weeks, 1-3 weeks, or 1-4 weeks), via parenteral, preferably via intravenously or intraarterially wherein each of the repeated doses is in turn administered by dividing such dose in two or more sub doses of about 1 ml of total volume each, and administering said two or more sub doses of 1 about ml of total volume each to the subject sequentially until the total dose is completely administered, preferably in a total period of time of from 5 to 10 minutes.

Preferably, the first aspect of the invention refers to a pharmaceutical composition comprising an effective amount of isolated human mesenchymal stem cells (MSCs) in an acceptable pharmaceutical liquid medium for injection, for use in a method of preventing or treating a human subject having or at risk of developing bronchopulmonary dysplasia or its sequelae, wherein the human subject is less than 37 weeks of gestation, preferably the human subject is born before 37 weeks of gestation, including those born at even shorter gestational terms (e.g., before 36, before 35, before 34, before 33, before 32, before 31, before 30, before 29, before 28, before 27, before 26, or before 25 weeks of gestation is less than four weeks old), and wherein the pharmaceutical composition is administered **in repeated doses** via the intravenous administration. Preferably, each of the repeated doses of the pharmaceutical composition is of about 5 × 10⁶ cells/kg and each repeated dose (of about 5 × 10⁶ cells/kg) is in turn sequentially administered to the patient **in repeated sub-doses** intravenously of about one million MSCs/ml each by using pre-filled syringes of 1 ml each. Preferably, such repeated administration of **sub-doses** intravenously of about one million MSCs/ml each by using pre-filled syringes of 1 ml each, is performed or carried out by the administration of each of the pre-filled syringes sequentially over a period of several minutes, usually from 5 to 10 minutes, depending on the weight of the patient and thus on the number of pre-filled syringes to be administered to reach the amount of about 5 × 10⁶ cells/kg. Preferably, each pre-filled syringe is administered in time intervals of 1-2 minutes each for the full infusion of the contents of each pre-filled syringe. The pre-filled syringes are preferably thus injected successively, using a total time of between 5 and 10 minutes to inject all of the pre-filled syringes (the number of syringes and the total time needed to inject about 5 × 10⁶ cells/kg depends on the weight of the patient).

In a preferred embodiment, the human subject has a birth weight ≤1,250 grams and < 32 weeks gestational age, preferably <28 weeks gestational age (GA), and is at high risk of developing BPD, preferably the patient is on mechanical ventilation, preferably with a FiO₂ < 30%, more preferably with a FiO₂ ≥30%.

As used herein, the term "about" shall be understood as ± 30%, ± 20%, ± 10%, ± 5%,or ± 1% of the compound making reference to. For example, about 5 × 10⁶ cells/kg, as used herein, means any amount between 3.5× 10⁶ cells/kg and 6.5× 10⁶ cells/kg (± 30%), preferably any amount between 4 × 10⁶ cells/kg and 6 × 10⁶ cells/kg (± 20%), preferably any amount between 4.5× 10⁶ cells/kg and 5.5× 10⁶ cells/kg (± 10%), preferably any amount between 4.75× 10⁶ cells/kg and 5.25× 10⁶ cells/kg (± 5%), or preferably any amount between 4.95× 10⁶ cells/kg and 5.05× 10⁶ cells/kg (± 1%). The same is true for the term "about one million MSCs/ml" which shall be understood as any amount between 0.7× 10⁶ cells/kg and 1.3 × 10⁶ cells/kg (± 30%), preferably any amount between 0.8 × 10⁶ cells/kg and 1.2 × 10⁶ cells/kg (± 20%), preferably any amount between 0.9 × 10⁶ cells/kg and 1.1 × 10⁶ cells/kg (± 10%), preferably any amount between 0.95 × 10⁶ cells/kg and 1.05 × 10⁶ cells/kg (± 5%), or preferably any amount between 0.99 × 10⁶ cells/kg and 1.01 × 10⁶ cells/kg (± 1%).

As used herein, BPD sequelae shall be understood as one or more of the following:
- asthma, bronchial hyperreactivity, need for oxygen therapy, decreased lung function, recurrent respiratory infections, increased incidence of emphysema and COPD in adulthood;
- Death as a consequence of pre-maturity;
- other complications of prematurity such as death, necrotizing enterocolitis and its sequelae (need for digestive surgery with intestinal resection, and ostomy, short intestine, need for parenteral nutrition, liver disease derived from parenteral nutrition) neurological complications (intraventricular hemorrhage, periventricular leukomalacia, ), and neurological sequelae (spastic paresis, hydrocephalus, attention deficit hyperactivity disorder, hearing loss, decreased intelligence quotient), recurrent infections, patent ductus arteriosus, retinopathy of prematurity and its sequelae (decreased visual acuity, disorders ocular motility, strabismus) pulmonary hypertension, cardiovascular anomalies and their cardiovascular sequelae (ventricular dysfunction, increased incidence of ischemic heart disease, systemic arterial hypertension), metabolic disorders of prematurity (osteopenia, diabetes) and late metabolic complications (obesity d, insulin resistance), acute or chronic renal dysfunction.

The MSCs of the present invention are used (e.g., administered) in pharmaceutically acceptable preparations (or pharmaceutically acceptable compositions), typically when combined with a pharmaceutically acceptable carrier. Such preparations may routinely contain pharmaceutically acceptable concentrations of salt, buffering agents, preservatives, compatible carriers, and may optionally comprise other (i.e., secondary) therapeutic agents.

A pharmaceutically acceptable carrier is a pharmaceutically acceptable material, composition or vehicle, such as a liquid or solid filler, diluent, excipient, solvent or encapsulating material, involved in carrying or transporting a prophylactically or therapeutically active agent. Each carrier must be "acceptable" in the sense of being compatible with the other ingredients of the formulation and not injurious to the subject. Some examples of materials which can serve as pharmaceutically acceptable carriers include sugars, such as lactose, glucose and sucrose; glycols, such as propylene glycol; polyols, such as glycerin, sorbitol, mannitol and polyethylene glycol; esters, such as ethyl oleate and ethyl laurate; buffering agents, such as magnesium hydroxide and aluminum hydroxide; pyrogen-free water; isotonic saline; Ringer's solution; ethyl alcohol; phosphate buffer solutions; and other nontoxic compatible substances employed in pharmaceutical formulations.

Effective Amounts. The preparations of the invention are administered in effective amounts. An effective amount is that amount of an agent that alone stimulates the desired outcome. The absolute amount will depend upon a variety of factors, including the material selected for administration, whether the administration is in single or multiple doses, and individual patient parameters including age, physical condition, size, weight, and the stage of the disease. These factors are well known to those of ordinary skill in the art and can be addressed with no more than routine experimentation. In particular, an effective amount is preferably understood as repeated doses (including two, three, four, five or more administrations of MSCs) of about 5 × 10⁶ cells/kg, more preferably about 2 to 4 doses of about 5 × 10⁶ cells/kg, more preferably three doses of about 5 × 10⁶ cells/kg.

A mesenchymal stem cell, according to the present invention, is a progenitor cell having the capacity to differentiate into neuronal cells, adipocytes, chondrocytes, osteoblasts, myocytes, cardiac tissue, and other endothelial and epithelial cells. (See for example Wang, Stem Cells 2004;22(7); 1330-7; McElreavey;1991 Biochem Soc Trans (1);29s; Takechi, Placenta 1993 March/April; 14 (2); 235-45; Takechi, 1993; Kobayashi; Early Human Development; 1998; July 10; 51 (3); 223-33; Yen; Stem Cells; 2005; 23 (1) 3-9.) These cells may be defined phenotypically by gene or protein expression. These cells have been characterized to express (and thus be positive for) one or more of CD13, CD29, CD44, CD49a, b, c, e, f, CD51, CD54, CD58, CD71, CD73, CD90, CD102, CD105, CD106, CDw119, CD120a, CD120b, CD123, CD124, CD126, CD127, CD140a, CD166, P75, TGF-bIR, TGF-bIIR, HLA-A, B, C, SSEA-3, SSEA-4, D7 and PD-L1. These cells have also been characterized as not expressing (and thus being negative for) CD3, CD5, CD6, CD9, CD10, CD11a CD14, CD15, CD18, CD21, CD25, CD31, CD34, CD36, CD38, CD45, CD49d, CD50, CD62E, L, S, CD80, CD86, CD95, CD117, CD133, SSEA-1, and ABO. Thus, mesenchymal stem cells may be characterized phenotypically and/or functionally according to their differentiative potential.

Mesenchymal stem cells may be harvested from a number of sources including but not limited to bone marrow, blood, periosteum, dermis, umbilical cord blood and/or matrix (e.g., Wharton's Jelly), and placenta. The MSCs of the present invention are preferably derived from Wharton's Jelly.

The mesenchymal stem cells contemplated for use in the methods of the disclosure may be derived from the same subject to be treated (and therefore would be referred to as autologous to the subject) or they may be derived from a different subject preferably of the same species (and therefore would be referred to as allogeneic to the subject).

As used herein, it is to be understood that aspects and embodiments of the invention relate to cells as well as cell populations, unless otherwise indicated. Thus, where a cell is recited, it is to be understood that a cell population is also contemplated unless otherwise indicated.

As used herein, an isolated mesenchymal stem cell is a mesenchymal stem cell that has been physically separated from its natural environment, including physical separation from one or more components of its natural environment. Thus, an isolated cell or cell population embraces a cell or a cell population that has been manipulated in vitro or ex vivo. As an example, isolated mesenchymal stem cells may be mesenchymal stem cells that have been physically separated from at least 50%, preferably at least 60%, more preferably at least 70%, and even more preferably a least 80% of the cells in the tissue from which the mesenchymal stem cells are harvested. In some instances, the isolated mesenchymal stem cells are present in a population that is at least 40%, at least 50%, at least 60%, at least 70%, at least 80%, at least 85%, at least 90%, at least 95%, at least 96%, at least 97%, at least 98%, at least 99%, or 100% mesenchymal stem cells as phenotypically and/or functionally defined herein. Preferably the ratio of mesenchymal stem cells to other cells is increased in the isolated preparation as compared to the starting population of cells.

Mesenchymal stem cells can be isolated using methods known in the art, e.g., from bone marrow mononuclear cells, umbilical cord blood, adipose tissue, placental tissue, based on their adherence to tissue culture plastic. For example, mesenchymal stem cells can be isolated from commercially available bone marrow aspirates. Enrichment of mesenchymal stem cells within a population of cells can be achieved using methods known in the art including but not limited to FACS.

Commercially available media may be used for the growth, culture and maintenance of mesenchymal stem cells. Such media include but are not limited to Dulbecco's modified Eagle's medium (DMEM). Components in such media that are useful for the growth, culture and maintenance of mesenchymal stem cells include but are not limited to amino acids, vitamins, a carbon source (natural and non-natural), salts, sugars, plant derived hydrolysates, sodium pyruvate, surfactants, ammonia, lipids, hormones or growth factors, buffers, non-natural amino acids, sugar precursors, indicators, nucleosides and/or nucleotides, butyrate or organics, DMSO, animal derived products, gene inducers, non-natural sugars, regulators of intracellular pH, betaine or osmoprotectant, trace elements, minerals, non-natural vitamins. Additional components that can be used to supplement a commercially available tissue culture medium include, for example, animal serum (e.g., fetal bovine serum (FBS), fetal calf serum (FCS), horse serum (HS)), antibiotics (e.g., including but not limited to, penicillin, streptomycin, neomycin sulfate, amphotericin B, blasticidin, chloramphenicol, amoxicillin, bacitracin, bleomycin, cephalosporin, chlortetracycline, zeocin, and puromycin), and glutamine (e.g., L-glutamine). Mesenchymal stem cell survival and growth also depends on the maintenance of an appropriate aerobic environment, pH, and temperature. Mesenchymal stem cells can be maintained using methods known in the art. (See for example Pittenger et al., Science, 284:143-147 (1999).)

On the other hand, the methods of the disclosure, although they may be performed on any subject likely to derive benefit therefrom, including human subjects, agricultural livestock (e.g., cows, pigs, etc.), prized animals (e.g., horses), companion animals (e.g., dogs, cats, etc.), and the like, preferably the present invention is directed to human subjects. The subjects may be those that have a lung disease (or condition) amenable to treatment using the MSCs of the invention, or they may be those that are at risk of developing such a disease (or condition). Such subjects include neonates and particularly neonates born at low gestational age. As used herein, a human neonate refers to a human from the time of birth to about 4 weeks of age. As used herein, a human infant refers to a human from about the age of 4 weeks of age to about 3 years of age. As used herein, low gestational age refers to birth (or delivery) that occurs before a normal gestational term for a given species. In humans, a full gestational term is about 40 weeks and may range from 37 weeks to more than 40 weeks. Low gestational age, in humans, akin to a premature birth is defined as birth that occurs before 37 weeks of gestation. The invention therefore contemplates prevention and/or treatment of human subjects born before 37 weeks of gestation, including those born at even shorter gestational terms (e.g., before 36, before 35, before 34, before 33, before 32, before 31, before 30, before 29, before 28, before 27, before 26, or before 25 weeks of gestation). Typically, such premature infants will be treated as neonates, however the invention contemplates their treatment even beyond the neonate stage and into childhood and/or adulthood. Certain subjects may have a genetic predisposition to certain forms of lung disease such as for example pulmonary hypertension, and those subjects may also be treated according to the invention.

The invention contemplates preventing and treating Bronchopulmonary dysplasia or its sequelae. Preventing a disease means reducing the likelihood that the disease manifests itself and/or delaying the onset of the disease. Treating a disease means reducing or eliminating the symptoms or sequelae of the disease.

Bronchopulmonary dysplasia is a condition that afflicts neonates who have been given oxygen or have been on ventilators, or neonates born prematurely particularly those born very prematurely (e.g., those born before 32 weeks of gestation). It is also referred to as neonatal chronic lung disease. Causes of BPD include mechanical injury for example as a result of ventilation, oxygen toxicity for example as a result of oxygen therapy, and infection. The disease may progress from non-inflammatory to inflammatory with time. Symptoms include bluish skin, chronic cough, rapid breathing, and shortness of breath. Subjects having BPD are more susceptible to infections such as respiratory syncytial virus infection. Subjects having BPD may develop pulmonary hypertension.

Prevention and/or treatment may involve in some instances use of the MSC alone or together with one or more secondary agents. Subjects may also be subjected to mechanical interventions such as ventilation with or without exogenous oxygen administration.

With respect to neonates and particularly low gestation age neonates, the invention contemplates administration of MSC within 4 weeks, 3 weeks, 2 weeks, 1 week, 6 days, 5 days, 4 days, 3 days, 2 days, 1 day, 12 hours, 6 hours, 3 hours, or 1 hour of birth. In some instances, the MSC are administered within 1 hour of birth.

The disclosure further contemplates administration of MSC even in the absence of symptoms indicative of BPD.

The disclosure contemplates repeated administration of MSC, including two, three, four, five or more administrations of MSCs. In some instances, the MSCs may be administered continuously. Repeated or continuous administration may occur over a period of several hours (e.g., 1-2, 1-3, 1-6, 1-12, 1-18, or 1-24 hours), several days (e.g., 1-2, 1-3, 1-4, 1-5, 1-6 days, or 1-7 days) or several weeks (e.g., 1-2 weeks, 1-3 weeks, or 1-4 weeks) depending on the severity of the condition being treated. If administration is repeated but not continuous, the time in between administrations may be hours (e.g., 4 hours, 6 hours, or 12 hours), days (e.g., 1 day, 2 days, 3 days, 4 days, 5 days, or 6 days), or weeks (e.g., 1 week, 2 weeks, 3 weeks, or 4 weeks). The time between administrations may be the same or they may differ. As an example, if the symptoms of the disease appear to be worsening the MSC may be administered more frequently, and then once the symptoms are stabilized or diminishing the MSC may be administered less frequently.

In some instances, the pharmaceutical composition is administered in three doses, wherein the first dose is administered to a human subject less than four weeks of age, wherein the second dose is administered after 2-14 days after the first dose and the third dose is administered after 2-14 days after the second dose.

In some instances, the pharmaceutical composition is administered in three doses, wherein the first dose is administered to a human subject less than four weeks of age, wherein the second dose is administered after 5-9 days after the first dose and the third dose is administered after 5-9 days after the second dose.

In some instances, the first dose of the repeated doses is administered at any time between 7 to 21 postnatal days.

In some instances, the mesenchymal stem cells are cultured, genetically unmanipulated mesenchymal stem cells isolated from umbilical cord.

In some instances, each dose of the repeated doses of the pharmaceutical composition is administered in an amount of from about 1×10⁵ cells/kg to about 1×10⁷ cells/kg. Preferably, each dose of the pharmaceutical composition is administered in an amount of from about 3×10⁶ cells/kg to about 7×10⁶ cells/kg. More preferably, each dose of the pharmaceutical composition is administered in an amount of about 5×10⁶ cells/kg (as defined above), wherein the mesenchymal stem cells are umbilical cord mesenchymal stem cells, and wherein the pharmaceutical composition is preferably administered in three doses, wherein the first dose is administered to a human subject less than four weeks of age, wherein the second dose is administered after 2- 14 days after the first dose and the third dose is administered after 2- 14 days after the second dose. Still more preferably, each dose of the pharmaceutical composition is administered in an amount of about 5×10⁶ cells/kg, wherein the mesenchymal stem cells are umbilical cord mesenchymal stem cells, and wherein the pharmaceutical composition is administered in three doses, wherein the first dose is administered to a human subject less than four weeks of age, wherein the second dose is administered after 5- 9 days after the first dose and the third dose is administered after 5- 9 days after the second dose. Still more preferably, each dose of the pharmaceutical composition is administered in an amount of about 5×10⁶ cells/kg, wherein the mesenchymal stem cells are umbilical cord mesenchymal stem cells, and wherein the pharmaceutical composition is administered in three doses, wherein the first dose is administered to a human subject at any time between 7 to 21 postnatal days, wherein the second dose is administered after 5- 9 days after the first dose and the third dose is administered after 5- 9 days after the second dose.

In some further instances, the repeated doses increased production or secretion of an anti-inflammatory cytokines interleukin-4 or interleukin-10. In still some further instances, the repeated doses of mesenchymal stem cells significantly modify the expression in a blood, serum or plasma sample isolated from the subject, after one, two or three of the doses, of one or more of the following miRNAs hsa-miR-598-3p, hsa-miR-193b-5p and hsa-miR-675-3p, in comparison to a control or reference sample. Preferably, in the context of the use of three doses, the repeated doses provide for a significant under expression of hsa-miR-103a-3p, hsa-miR-107-5p, hsa-miR-30b-5p, and hsa-miR-32-5p (> 2-fold value) in comparison to control untreated samples, in blood samples taken from the ELGAN infants after administering the second and after administering a third dose of the repeated doses of the pharmaceutical composition. Preferably, the repeated doses provide for a significant under expression of hsa-miR-598-3p (> 2-fold value) in comparison to control untreated samples, in blood samples taken from the ELGAN infants after administering the third dose. Preferably, in the context of the use of three doses, the repeated doses provide for a significant over expression of hsa-miR-193b-5p and hsa-miR-675-5p, (> 2-fold value) in comparison to control untreated samples, in blood samples taken from the ELGAN infants after administering the second and after administering the third dose. Such inhibition or increased expression of the indicated miRNAs is indicative of good prognosis.

Finally, the present invention also refers to a packaged and labelled pharmaceutical product containing one or more doses of the pharmaceutical composition of the invention, wherein each dose is in turn composed or made up of two or more sub doses wherein each sub dose is in turn contained or comprised in pre-filled syringes of 1 ml each. Preferably, each dose of about 5 × 10⁶ cells/kg is thus made up of repeated **sub doses** of about one million MSCs/ml each by using pre-filled syringes of 1 ml each. This article of manufacture or kit includes the appropriate unit of sub dosage form in an appropriate vessel or container such as a glass vial or plastic ampoule or other container that is hermetically sealed, preferably each unit is a pre-filled syringe of 1 mL. The unit dosage form should be suitable for intravenous delivery. Preferably, the article of manufacture or kit further comprises instructions on how to use including how to administer the pharmaceutical product. The instructions may further contain informational material that advises a medical practitioner, technician or subject on how to appropriately prevent or treat the disease or disorder in question. In other words, the article of manufacture includes instructions indicating or suggesting a dosing regimen for use including but not limited to actual doses, monitoring procedures, and other monitoring information.

As with any pharmaceutical product, the packaging material and container are designed to protect the stability of the product during storage and shipment.

The kits may include MSC in sterile aqueous suspensions that may be used directly or may be diluted with normal saline for intravenous injection. The kits may therefore also contain the diluent solution or agent, such as saline.

### Brief description of the figures

**Fig. 1****.** Scheme of UC-MSCs administration.
**Fig. 2** Evolution of the RSS score and ratio of proinflammatory (IL-6) and antiinflamatory (IL-10) interleukins after the MSCs administration. **Left:** RSS and ratio of IL-6/Il-10 in serum. **Right:** RSS and ratio of IL-6/Il-10 in tracheal aspirates. +14: (pre-MSCs administration), +21: one week after the first MSCs dose, and previous to the second MSCs dose; +28: one week before the third MScs dose; + 28: one week after the third and last MScs administration.

### Examples

### Example 1

### 1. PATIENTS AND METHODS

### Study design and objectives

We designed a phase I, open-label, single-arm, multicenter clinical trial to evaluate the feasibility and safety of intravenous allograft transplantation of UC-MSCs in ELGAN infants at high risk of BPD, under the acronym PULMESCELL.

The target sample size was 10 patients. Patients were enrolled at four different hospitals pertaining to the Spanish Public National Health System: Hospital Universitario La Paz (Madrid), Hospital Universitario San Carlos (Madrid), Hospital Universitari i Politècnic La Fe (Valencia), and Complexo Hospitalario Universitario A Coruña (A Coruña). Patients were recruited between April 2019 and June 2020.

The secondary goal was to evaluate the changes in inflammatory, oxidative stress and miRNA profile after the MSCs administration.

We have reported the adverse events occurred during the Pulmescell trial and retrospectively compared them with complications present in a cohort of ELGAN infants with similar clinical characteristics who were previously assisted in the same Neonatology Units participating in the trial and two additional units with similar protocols. Only patients with complete data were included in this comparison group. A total of 20 patients met the criteria and were included in this comparison group.

The AEMPS (Spanish Medicine and Health Products Regulatory Agency) and SCReN (Spanish Clinical Research Network) acted as external monitors.

### inclusion and exclusion criteria

Inclusion and exclusion criteria are shown in Table 1.

**Table 1: inclusion and exclusion criteria**

| **Inclusion criteria** | **Exclusion criteria** |
|---|---|
| GA < 28 weeks | Severe congenital anomalies** and chromosomopathies |
| Birth weight ≤1250 g | Severe septic shock or hemodynamic instability |
| IMV with FiO₂ > 0.3 at postnatal day 7-14* | Severe intraventricular hemorrhage (grade ≥3 according to Volpe classification (15)) |
| | Necrotizing enterocolitis (grade >II according to Bell classification (16)) |
| | Major surgery in the 72 h before inclusion |
| | Active pulmonary bleeding |
| | HIV infection |

| | |
|---|---|
| *During the first six months, we enrolled patients on IMV at postnatal day 14. Due to difficulties in recruitment and according to the results of Hunt KA et al. (14), we got the approval of the AEMPS for a protocol amendement, allowing the inclusion of ELGAN on IMV with FiO₂ >0.3 from day 7^{th}. **Severe congenital anomalies, including lung hypoplasia, renal malformations, congenital heart disease and multiple malformative syndromes. | |

### Definitions

BPD was defined according to the National Institutes of Health workshop severity-based diagnostic criteria (table 2) (17). In the treatment group, we also reclassified patients according to Jensen's definition of BPD (18), as this classification has been shown to correlate with health outcomes (19).

**Table 2. Definition of bronchopulmonary dysplasia: diagnostic criteria. BPD = bronchopulmonary dysplasia; D= day; NCPAP = nasal continuous positive airway pressure; PMA = postmenstrual age; PPV = positive-pressure ventilation; wk= week.**

| **Gestational age** | **< 32 wk** | **≥ 32 wk** |
|---|---|---|
| **Time-point assessment** | 36 wk PMA or discharge | >28-56 d postnatal age or discharge |

| **Treatment with oxygen FiO₂ > 0.21 for at least 28 d and** | | |
|---|---|---|
| **Mild BPD** | Breathing room air | |
| **Moderate BPD** | FiO₂ < 0.3 | |
| **Severe BPD** | FiO₂ ≥ 30% and/or positive pressure (PPV or NCPAP) | |

| | | |
|---|---|---|
| Jobe AH, Bancalari E. Bronchopulmonary dysplasia. Am J Respir Crit Care Med. 2001;163:1723-9. | | |

We assessed a simplified version of the respiratory severity score (RSS), consisting in the mean airway pressure (MAP) multiplied by the fraction of inspired oxygen (FiO2) (20), before and one week after each UC-MSCs administration.

### Production and administration of the cell therapy

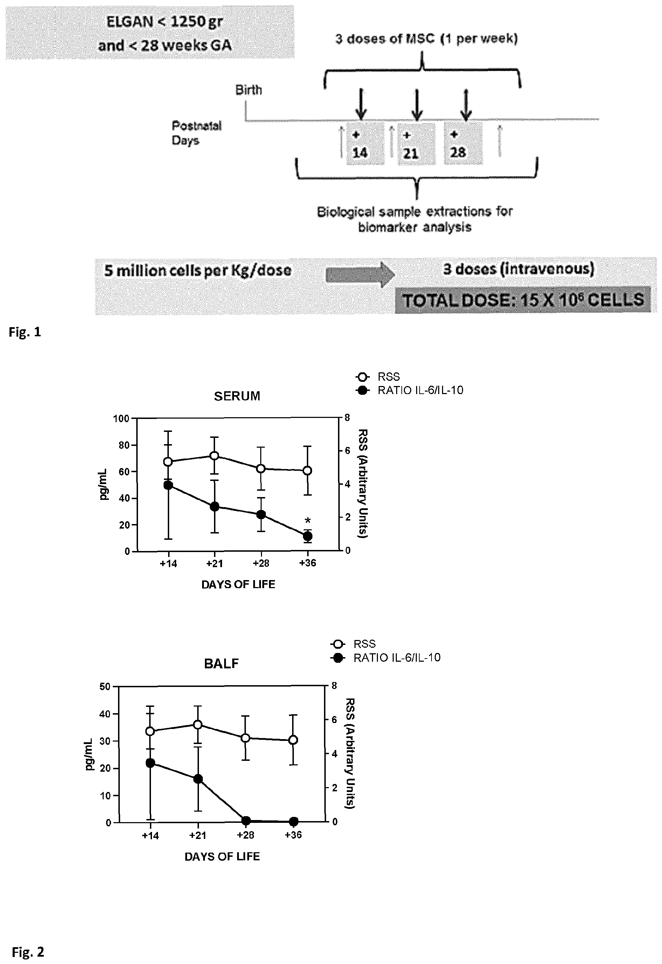

All patients were assigned to receive 3 doses of UC-MSCs (5 × 10⁶ cells/kg), one per week (figure 1).

UC-MSCs were prepared in compliance with good manufacturing practices, at Niño Jesús Gene and Cell Therapy Laboratory of Hospital Universitario Niño Jesús (Madrid), accredited by the AEMPS to produce UC-MSCs for human use. UC-MSCs were extracted from Wharton's jelly, expanded, and frozen. Once a patient was recruited, the laboratory defrosted the MSCs and released the dose according to the patient's weight in sterile syringes ready to be injected. The vials were sent to the recruiting NICUs using a specialized transport company, with control and registration of the temperature of the vial (data logger) throughout the entire transport.

The syringes were gently turned over several times in order to avoid cell aggregates just before the injection and were administered directly through a peripheral vein access (figure 1).

In particular, the UC-MSCs was produced according to Good Manufacturing Practices at Unidad de Fabricación de Medicamentos de Terapia Avanzada-Hospital Infantil Universitario Niño Jesus (UFMTA, Madrid, Spain). Quality control tests were conducted in accordance to European Pharmacopeia standards, when applicable. The production Unit is authorized for the manufacturing of this medicine by the local regulatory authority (Agencia Española de Medicamentos y Productos Sanitarios, AEM PS). Starting material (GMP hWJ-derived MSCs in p2 (human Wharton Jelly derived Mesenchymal Stem Cells) was obtained from Polski Bank Komórek Macierzystych S.A. (Warshaw, Poland). Cells were isolated from umbilical cord fragments taken from single donor healthy full-term mother, after obtaining informed consent. Donor-mother's health status was based on the medical questionnaire and testing of infectious agents in a sample of her peripheral blood collected on the birthday according to local regulations. Briefly, hWJ was collected and processed within 24 hours. After disinfected and cut into pieces of 0.5-1 mm² fragments were cultivated in a serum-free, xeno-free system with 0.25 µg/ml Amphotericin B, 100 U/ml Penicillin, 100 µg/ml Streptomycin. After 48 days, cells were cryopreserved in saline, 10% DMSO, 5% human albumin and transport to UFMTA.

Cells were then thaw and expanded in DMEM (1 gr/ml glucose, 110 mM pyruvate, GIBCO, UK) supplemented with 10% γ-irradiated FBS (Gibco, Australia) in order to manufacture a cryopreserved working cell bank (Active Substance). When ≥100×10⁶ cell count was reached (≤8 population doublings), cells were cryopreserved in γ-irradiated FBS, 10% DMSO. MSCs were characterized by FACS confirming the expression of CD90, CD73, and CD29 surface molecules (≥85% of viable cells) and the absence of CD14, CD19, and CD45 (≤5% of viable cells). Cells were maintained at -196ºC until needed.

In preparation for administration, frozen MSCs were thawed and recovered 5-7 days in DMEM (1 gr/ml glucose, 110 mM pyruvate, GIBCO, UK) supplemented with 10% γ-irradiated FBS (Gibco, Australia). Cells were trypsinized, counted and conditioned in 2% human albumin (Grifols, Spain) in saline (B Braun, Spain). Cells were packed at 1.0×10⁶ cells/ml in three to eight (according to patient's weight) 1-ml sterile syringes with cap, labelled and released for immediate administration. Transportation was at 15-25ºC, with a shelf life of 24 hours from the time of manufacture.

The battery of QC tests included sterility, mycoplasma, endotoxins, cell viability and cell immunophenotype.

### Assessment of safety

Safety was defined primarily as the absence of treatment-related serious adverse events (SAEs) according to the Consolidated Standards of Reporting Trials (21), and secondarily as the absence of dose-related toxicity, defined as death within 6 hours after UC-MSC transplantation or anaphylactic shock related to the UC-MSC injection. After each intravenous UC-MSC injection, all patients were regularly and intensively monitored. Blood, urine, and tracheal aspirate samples were obtained before and 7 days after each UC-MSC administration to analyze miRNA profile, oxidative stress and inflammation biomarkers and to assess safety. Patients were followed up until 2 years of age for comparison of adverse outcomes for further safety evaluation.

### Study variables

Clinical, echocardiographic and analytical variables were recorded on an online database. We recorded the Respiratory Severity Score (RSS) of the included patients, from birth to 36 weeks GA.

### Statistical Analysis

After completing the trial, we compared the outcomes of the patients treated with UC-MSCs with the comparison cohort.

Data were collected prospectively from medical records. Categorical variables are expressed as frequencies and percentages and that of continuous variables as mean ± standard deviation (SD) or median (interquartile range).

### 2. RESULTS

From April 2019 to July 2020, 10 patients (6 males, 4 females) were recruited, with mean GA of 25 weeks (SD: 1.00, range 24-26) and birth weight of 660 grams (SD: 154, range 492-780).

All patients received per protocol 3 UC-MSC doses of 5×10⁶ cells/kg. The first UC-MSC dose was administered at the mean postnatal day 16 (SD: 2.4, range 13.6-18.4). No deaths were reported during the treatment period or at follow up. All patients were discharged from the hospital. No adverse effects related with the UC-MSCs administration were observed during administration or at 21-month (SD: 7.2) follow up.

Perinatal data of the treated patients and the patients in the comparison group is shown in table 3. There were no significant differences between both cohorts in GA and birth weight.

**Table 3. Basal perinatal characteristics of the patients recruited in the trial and in the control group. IUGR: Intrauterine growth retardation. MV: Mechanical Ventilation. RSS: respiratory severity score (mean airway pressure x FiO2)**

| | PULMESCELL (n=10) (%) Med±Sd (Q1-Q3) | Comparison group (n=20) (%) Med±Sd (Q1-Q3) |
|---|---|---|
| Male Gender | 60% | 65% |
| Gestational Age (GA, weeks) | 25±1 (24-25,2) | 24,81 |
| Birth Weight (grams) | 660 ±154 (492-780) | 711,28+125 |
| IUGR (P<3) | 10% | 0% |
| Spontaneous gestation | 80% | 70% |
| Single gestation | 50% | 75% |
| Mother's age | 35±7 (30-39) | 34,25±6 |
| Oligoamnios | 20% | 5% |
| Prenatal steroids | 100% | 100% |
| Complete prenatal steroids protocol | 30% | 55% |
| Chorioamnionitis pathology | 30% | 45% |
| intubation and MV at birth | 60% | 80% |
| SNAPPE-II | 51 ±17 (38-69) | 42,3 + 19,3 |
| Surfactant administration *(Number of doses)* | 100% | 100% |
| | 1 dose 50 % | 1 dose 60 % |
| | 2 doses 20% | 2 doses 30% |
| | 3 doses 30% | 3 doses 10% |
| RSS day 14 | 5,4 ±3,3 (2,3-6.7) | 5,5 ±3,1 (3-7,8) |

The respiratory situation and outcomes of the patients included in the trial and its comparison with the control group is shown in table 4. All patients in both cohorts were diagnosed with BPD according to the 2001 NIH definition (17), with similar distribution of severity.

**Table 4. Outcomes of the patients included in the trial and of the comparison group. MV: Mechanical ventilation. CPAP: Continuous positive airway pressure. BPD: Bronchopulmonary dysplasia. GA: Gestational Age. NC: Nasal cannula. HFNC: High Flow nasal cannula.**

| | PULMESCELL (n=10) (%) Med±Sd (Q1-Q3) | Comparison group (n=20) (%) Med±Sd (Q1-Q3) |
|---|---|---|
| **BPD^{∗}** *(^{∗}Bancalari definition)* | 100% | 100% |
| **BPD Degree*** *(^{∗}Bancalari definition)* | Mild, n=1/10 | Mild, n=1/18** |
| | Moderate, n=4/10 | Moderate, n=10/18 |
| | Severe, n=5/10 | Severe, n=7/18 |
| | | *..(2 deaths before 36 weeks PMA)* |
| **BPD** *(Jensen classification)* | NO BPD, n=1 | Not available (retrospective data) |
| | NO BPD, n=1 | |
| | Grade 1, n=1 | |
| | Grade 2, n=7 | |
| | Grade 3, n=1 | |
| **Death before discharge** | 0/10 | 3/20 (15%) |
| | | **2 deaths before 36 weeks PMA* |
| | | **1 death after 36 weeks, before discharge* |
| **BPD* or Death before discharge** *(*Bancalari definition)* | 10/10 | 0120 |
| **Death or Severe DBP*** *(*Bancatari definition)* | 5/10 | 9/20 |
| **Postnatal steroids** | 90% (80% during MSCs administration) | 90% |
| **Respiratory condition at 36 w GA** | Room Air n=1 | Room air n=1 |
| | O2 NC n=1 | O2 NC n= 7 |
| | HFNC n=4 | HFNC n=6 |
| | NIV n=4 | NIV n=4 *(n=18: 2 deaths before* 36 *weeks PMA)* |
| **Supplemental Oxygen at discharge (*survivors at discharge)** | 6/10 | 11/17* |
| | | **2 deaths before 36 weeks PMA* |
| | | **1 death after 36 weeks, before discharge* |

The incidence of prematurity-related morbidities was similar to that observed in the comparison group (table 5).

**Table 5. Comparison of prematurity related complications in the trial patients and in the comparison group. NEC: Necrotizing enterocolitis. ROP: Retinopathy of prematurity. IHV: Intraventricular hemorrhage. PDA (Persistent Ductus Arteriosus)**

| | **PULMESCELL (%) (N=10)** | | **COMPARISON GROUP (N=20)** |
|---|---|---|---|
| **NEC** | 10% | | 15% |
| **Nosocomial Sepsis** | 80% | | 85% |
| **IHV** | | IHV > II 10% | IHV > II 10% |
| **ROP** | 70% | | 85% |
| **(Degree)** | I 1 patient | | I 3 patients |
| | II 1 patients | | II 2 patients |
| | III 3 patients | | III 10 patients |
| | > III 2 patients | | > III 1 patient |
| **ROP treatment** | 50% | 57,9% | |
| **PDA** | 90% | 80% | |
| **PDA Medical Treatment** | 90% | 75% | |
| **Surgical Or Percutaneous PDA Closure** | 50% | 45% | |

### Cell infusion

Intravenous transplantation of UC-MSCs took <10 minutes in all cases. All patients tolerated the procedure without complications within 24 hours after transplantation and no respiratory or cardiovascular compromise was observed.

### SAEs and prematurity related morbidities

Details of SAEs and prematurity-related morbidities, recorded along the 2 years after UC-MSC transplantation, are presented in Table 5. The 10 infants who received UC-MSC therapy were discharged alive. Postnatal corticosteroid use was similar in the UC-MSC transplantation cohort and in the comparison group. Interestingly, 8/10 patients treated with UC-MSCs received systemic corticosteroids during the UC-MSCs treatment period. No adverse effects related to this combination were observed.

All adverse events observed until 40 weeks PMA were registered and considered by the neonatologists in charge related to prematurity itself and not related with the UC-MSCs transfusion. The most common event was the need for patent ductus arteriosus (PDA) ligation, occurring in 5 of the 10 patients (50%). In the 2 years follow up, one patient born at 24 weeks GA who had received UC-MSCs, was diagnosed of spastic paresis at 18 months, but was reported as "not related" with the intervention. The incidence of this SAE in the cohort of treated patients was similar to that reported in the literature for ELGAN (22).

### Inflammatory biomarkers

Blood, urine, and tracheal aspirate samples were obtained before and a week after each UC-MSC administration to analyze inflammation biomarkers, miRNA profile and to assess safety.

Mean RSS before the first UC-MSC administration was 5.4 (SD: 3.3, range: 2.3-6.7). A trend towards improvement in the RSS score was observed after administration of UC-MCS, which was more evident after the first UC-MSC transplantation (fig. 2)

Likewise, a trend towards a decrease in the ratio of IL 6 to IL 10 ratio, was observed in both serum and tracheal aspirates after UC-MSC injection, suggesting a reduction in both pulmonary and systemic inflammation (fig. 2).

A further characterization of the inflammatory biomarkers in serum and tracheal aspirates, markers of oxidative stress and the miRNAs expression profile after the UC-MSCs administration has been performed. In this sense, hsa-miR-103a-3p, hsa-miR-107-5p, hsa-miR-30b-5p, and hsa-miR-32-5p were significantly under expressed (> 2-fold value) in comparison to control untreated samples, in blood samples taken from the ELGAN infants after administering the second and after administering the third dose. In addition, hsa-miR-598-3p was significantly under expressed (> 2-fold value) in comparison to control untreated samples, in blood samples taken from the ELGAN infants after administering the third dose. Hsa-miR-193b-5p and hsa-miR-675-5p, were significantly over expressed (> 2-fold value) in comparison to control untreated samples, in blood samples taken from the ELGAN infants after administering the second and after administering the third dose.

### References

1.- Siffel C, Kistler KD, Lewis JFM, Sarda SP. Global incidence of bronchopulmonary displasia among extremely preterm infants: a systematic literature review. J Matern Fetal Neonatal Med. 2021;34:1721-1731.
2.- Sahni M, Bhandari V. Recent advances in understanding and management of bronchopulmonary dysplasia. F1000Res. 2020 Jul 14;9:F1000 Faculty Rev-703.
3.- Willis GR, Fernández-González A, Anastas J, Vitali SH, Liu X, Ericsson M et al. Mesenchymal Stromal Cell Exosomes Ameliorate Experimental Bronchopulmonary Dysplasia and Restore Lung Function through Macrophage Immunomodulation. Am J Respir Crit Care Med. 2018 Jan 1;197(1):104-116.
4.- Chaubey S, Thueson S, Ponnalagu D, Alam MA, Gheorghe CP, Aghai Z et al. Early gestational mesenchymal stem cell secretome attenuates experimental bronchopulmonary dysplasia in part via exosome-associated factor TSG-6. Stem Cell Res Ther. 2018 Jun 26;9(1):173.
5.- You J, Zhou O, Liu J, Zou W, Zhang L, Tian D et al. Human Umbilical Cord Mesenchymal Stem Cell-Derived Small Extracellular Vesicles Alleviate Lung Injury in Rat Model of Bronchopulmonary Dysplasia by Affecting Cell Survival and Angiogenesis. Stem Cells Dev. 2020 Dec 1;29(23):1520-1532.
6.- Willis GR, Reis M, Gheinani AH, Fernández-González A, Taglauer ES, Yeung V et al. Extracellular Vesicles Protect the Neonatal Lung from Hyperoxic Injury through the Epigenetic and Transcriptomic Reprogramming of Myeloid Cells. Am J Respir Crit Care Med. 2021 Dec 15;204(12):1418-1432.
7.- Leeman KT, Pessina P, Lee JH, Kim CF. Mesenchymal Stem Cells Increase Alveolar Differentiation in Lung Progenitor Organoid Cultures. Sci Rep. 2019 Apr 23;9(1):6479.
8.- Möbius MA, Freund D, Vadivel A, Koss S, McConaghy S, Ohls RK et al. Oxygen Disrupts Human Fetal Lung Mesenchymal Cells. Implications for Bronchopulmonary Dysplasia. Am J Respir Cell Mol Biol. 2019 May;60(5):592-600.
9.- O'Reilly M, Möbius MA, Vadivel A, lonescu L, Fung M, Eaton F et al. Late Rescue Therapy with Cord-Derived Mesenchymal Stromal Cells for Established Lung Injury in Experimental Bronchopulmonary Dysplasia. Stem Cells Dev. 2020 Mar 15;29(6):364-371.
10.- Thébaud B, Lalu M, Renesme L, Van Katwyk S, Presseau J, Thavorn K et al. Benefits and obstacles to cell therapy in neonates: The INCuBAToR (Innovative Neonatal Cellular Therapy for Bronchopulmonary Dysplasia: Accelerating Translation of Research). Stem Cells Transl Med 2021 Jul;10(7):968-975.
11.- Chang YS, Ahn SY, Yoo HS, Sung SI, Choi SJ, Oh WI and Park WS. Mesenchymal Stem Cells for Bronchopulmonary Dysplasia: Phase 1 Dose-Escalation Clinical Trial. J Pediatr. 2014 May;164(5):966-972.
12.-Ahn SY, Chang YS, Lee MH, Sung SI, Lee BS, Kim KS et al. Stem cells for bronchopulmonary dysplasia in preterm infants: A randomized controlled phase II trial. Stem Cells Transl Med. 2021 Aug;10(8):1129-1137.
13- Álvarez-Fuente M, Arruza L, López-Ortego P, Moreno L, Ramirez-Orellana M, Labrandero C et al. Off-label mesenchymal stromal cell treatment in two infants with severe bronchopulmonary dysplasia: clinical course and biomarkers profile. Cytotherapy. 2018 Nov;20(11):1337-1344.
14.- Hunt KA, Dassios T, Ali K and Greenough A. Prediction of bronchopulmonary dysplasia development. Arch Dis Child Fetal Neonatal Ed. 2018 Nov;103(6):F598-F599.
15.- Volpe, J.J. Perinatal brain injury: from pathogenesis to neuroprotection. Ment Retard Dev Disabil Res Rev. 2001;7(1):56-64.
16.- Gregory KE, Deforge CE, Natale KM, Phillips M and Van Marter LJ. Necrotizing enterocolitis in the premature infant: neonatal nursing assessment, disease pathogenesis, and clinical presentation. Adv Neonatal Care. 2011 Jun;11(3):155-64; quiz 165-6.
17.-Jobe AH, Bancalari E. Bronchopulmonary dysplasia. Am J Respir Crit Care Med. 2001;163:1723-9.
18.- Jensen EA, Dysart K, Gantz MG, McDonald S, Bamat NA, Keszler M et al. The diagnosis of bronchopulmonary dysplasia in very preterm infants. An evidence-based approach. Am J Respir Crit Care Med. 2019;200(6):751-759**.**
19.- Jensen EA, Edwards EM, Greenberg LT, Soll RF, Ehret DEY, Horbar JD. Severity of Bronchopulmonary Dysplasia Among Very Preterm Infants in the United States. Pediatrics, 2021 Jul;148(1):e2020030007.
20.- Jung YH, Jang J, Kim H-S, Shin SH, Choi CW, Kim E-K and Kim BI. Respiratory severity score as a predictive factor for severe bronchopulmonary dysplasia or death in extremely preterm infants. BMC Pediatr. 2019 Apr 23;19(1):121.
21.- Ioannidis JP, Evans SJ, Gotzsche PC, O'Neill RT, Altman DG, Schulz K et al. Better reporting of harms in randomized trials: an extension of the CONSORT statement. Ann Intern Med 2004;141:781-8.
22.- Agut T, Póo P, Launes C, Auffant M and Iriondo M. Incidence of cerebral palsy in a cohort of preterm infants with a gestational age of less than 28 weeks. An Pediatr (Barc). 2015 Jan;82(1):49-50.
23.-Ávila-Álvarez A, Zozaya C, Pértega-Díaz S, Sánchez-Luna M, Iriondo-Sanz M, Elorza MD, Garcia-Muñoz Rodrigo F; Spanish Neonatal Network SEN1500. Temporal trends in respiratory care and bronchopulmonary dysplasia in very preterm infants over a 10-year period in Spain. Arch Dis Child Fetal Neonatal Ed. 2022 Mar;107(2):143-149.
24.- Gambera S, del Cerro MJ, Álvarez-Fuente M, Prieto R, Melen Frajilich G, González Murillo A et al. Biodistribution of umbilical cord-derived Mesenchimal Stem Cells in a mouse model. 11th International Conference. Neonatal and Childhood Pulmonary Vascular Disease. April 19-21;2018.
25.- Moreira Á, Winter C, Joy J, Winter L, Jones M, Noronnha M et al. Intranasal delivery of human umbilical cord Wharton's jelly mesenchymal stromal cells restores lung alveolarization and vascularization in experimental bronchopulmonary dysplasia. STEM CELLS Transl Med. 2020;9:221-234.
26.- Álvarez-Fuente M, Moreno L, López-Ortego P, Arruza L, Ávila-Álvarez A, Muro M et al. Exploring clinical, echocardiographic and molecular biomarkers to predict bronchopulmonary displasia. PLoS One, 2019; 14(3), e0213210.
27.- Jung YH, Jang JJ, Kim HS, Shin SH, Choi CW, Kim EK and Kim BI. Respiratory severity score as a predictive factor for severe bronchopulmonary dysplasia or death in extremely preterm infants. BMC Pediatr. 2019 Apr 23;19(1):121.
28.- Abele AN, Taglauer ES, Almeda M, Wilson N, Abikoye A, Seedorf GJ et al. Antenatal mesenchymal stromal cell extracellular vesicle treatment preserves lung development in a model of bronchopulmonary dysplasia due to chorioamnionitis. Am J Physiol Lung Cell Mol Physiol 2022 Feb 1;322(2): L179-L190.
29.- Chang YS, Choi SJ, Ahn SY, Sung DK, Sung SI, Yoo HS et al. Timing of Umbilical Cord Blood Derived Mesenchymal Stem Cells Transplantation Determines Therapeutic Efficacy in the Neonatal Hyperoxic Lung Injury. PLoS One. 2013;8(1):e52419.

## Claims

1. A pharmaceutical composition comprising an effective amount of isolated human mesenchymal stem cells (MSC) in an acceptable pharmaceutical liquid medium for injection, for use in a method of preventing or treating a human subject having or at risk of developing bronchopulmonary dysplasia, wherein the human subject is less than four weeks old, has a birth weight ≤1,250 grams and <32 weeks gestational age (GA), preferably <28 weeks gestational age (GA), and is preferably on mechanical ventilation and wherein the pharmaceutical composition is administered intravenously to the subject in repeated doses over a period of several weeks (e.g., 1-2 weeks, 1-3 weeks, or 1-4 weeks), and wherein each of the repeated doses is in turn administered by dividing such dose in two or more sub doses of about 1 ml of total volume each, and administering said two or more sub doses of 1 about ml of total volume each to the subject sequentially until the total dose is completely administered, preferably in a total period of time of from 5 to 10 minutes

2. The pharmaceutical composition according to claim 1, wherein each of the sub doses is administered intravenously by using syringes having a total volume of the syringe of about 1.0 mL.

3. The pharmaceutical composition according to any one of claims 1 or 2, wherein the pharmaceutical composition is administered in two or more doses, and wherein the first dose is administered to a human subject less than four weeks of age.

4. The pharmaceutical composition according to any one of claims 1 or 2, wherein the pharmaceutical composition is administered in three doses, wherein the first dose is administered to a human subject less than four weeks of age, wherein the second dose is administered after 2-14 days after the first dose and the third dose is administered after 2-14 days after the second dose.

5. The pharmaceutical composition according to claim 2, wherein the pharmaceutical composition is administered in three doses, wherein the first dose is administered to a human subject less than four weeks of age, wherein the second dose is administered after 5-9 days after the first dose and the third dose is administered after 5-9 days after the second dose.

6. The pharmaceutical composition according to any one of claims 1 to 5, wherein the first dose of the repeated doses is administered at any time between 7 to 21 postnatal days.

7. The pharmaceutical composition according to any one of claims 1 to 6, wherein the mesenchymal stem cells are cultured, genetically unmanipulated mesenchymal stem cells isolated from the Wharton's gelatine of the human umbilical cord.

8. The pharmaceutical composition according to any one of claims 1 to 7, wherein each dose of the pharmaceutical composition is administered in an amount of from about 3×10⁶ cells/kg to about 7×10⁶ cells/kg, wherein each dose is is in turn administered by dividing such dose in multiple sub doses of about 1 ml of total volume each at a concentration of about one million MSCs/ml.

9. The pharmaceutical composition according to any one of claims 1 to 7, wherein each dose of the pharmaceutical composition is administered in an amount of from about 4×10⁶ cells/kg to about 6×10⁶ cells/kg, wherein each dose is is in turn administered by dividing such dose in multiple sub doses of about 1 ml of total volume each at a concentration of about one million MSCs/ml.

10. The pharmaceutical composition according to any one of claims 1 to 7, wherein each dose of the pharmaceutical composition is administered in an amount of +/-10% of 5×10⁶ cells/kg, wherein the mesenchymal stem cells are umbilical cord mesenchymal stem cells, and wherein the pharmaceutical composition is administered in three doses, wherein the first dose is administered to a human subject less than four weeks of age, wherein the second dose is administered after 2-14 days after the first dose and the third dose is administered after 2-14 days after the second dose.

11. The pharmaceutical composition according to any one of claims 1 to 7, wherein each dose of the pharmaceutical composition is administered in an amount of +/-10% of 5×10⁶ cells/kg, wherein the mesenchymal stem cells are umbilical cord mesenchymal stem cells, and wherein the pharmaceutical composition is administered in three doses, wherein the first dose is administered to a human subject less than four weeks of age, wherein the second dose is administered after 5- 9 days after the first dose and the third dose is administered after 5- 9 days after the second dose.

12. The pharmaceutical composition according to any one of claims 1 to 7, wherein each dose of the pharmaceutical composition is administered in an amount of +/-10% of 5×10⁶ cells/kg, wherein the mesenchymal stem cells are umbilical cord mesenchymal stem cells, and wherein the pharmaceutical composition is administered in three doses, wherein the first dose is administered to a human subject at any time between 7 to 21 postnatal days, wherein the second dose is administered after 5- 9 days after the first dose and the third dose is administered after 5- 9 days after the second dose.

13. The pharmaceutical composition according to any one of claims 1 to 12, wherein the pharmaceutical composition comprises a further active ingredient.

14. The pharmaceutical composition according to any one of claims 1 to 13, wherein the repeated doses increased production or secretion of an anti-inflammatory cytokines interleukin-4 or interleukin-10.

15. The pharmaceutical composition according to any one of claims 1 to 13, wherein the repeated doses of mesenchymal stem cells significantly modify the expression in a blood, serum or plasma sample isolated from the subject, after one, two or three of the doses, of one or more of the following miRNAs hsa-miR-598-3p, hsa-miR-193b-5p and hsa-miR-675-3p, in comparison to a control or reference sample.
